# EUROPEAN PATENT SPECIFICATION

(11) **EP 2 467 183 B1**
(45) Date of publication and mention of the grant of the patent: **08.01.2020**
(21) Application number: 10810604.8
(22) Date of filing: 19.08.2010
(51) Int. Cl.: A61M 5/32

(54) **PATIENT-CONTACT ACTIVATED NEEDLE STICK SAFETY DEVICE**
DURCH PATIENTENKONTAKT AKTIVIERTE NADELSTICH-SICHERHEITSVORRICHTUNG
DISPOSITIF DE SÉCURITÉ ACTIF CONTRE LES PIQÛRES D'AIGUILLE EN CONTACT AVEC UN PATIENT

(30) Priority: 19.08.2009 US 235278 P
(43) Date of publication of application: 27.06.2012
(73) Proprietor: Safety Syringes, Inc., Carlsbad, California 92010 (US)
(72) Inventor: FIELD, Frederick P., San Diego California 92130 (US); DOWDS, Philip, San Diego California 92129 (US); VERESPEJ, James M., San Marcos California 92078 (US)
(74) Representative: Brédeville, Odile Marie
(86) International application number: PCT/US2010/046031
(87) International publication number: WO 2011/022559

(56) References cited:
- WO-A1-2009/040602
- US-A- 4 894 055
- US-A- 5 964 731
- US-B1- 6 830 560
- US-B1- 7 001 364

## Description

The following describes a device that is used in conjunction with a needle-based medication injection device (e.g. a prefilled syringe) that prevents needle stick injuries after the medication has been injected into a patient. The used needle is shielded by a cylindrical needle guard that surrounds and extends beyond the needle tip. In a preferred embodiment, before the needle is inserted into the patient, the needle guard projects forward to substantially hide visibility of the needle to reduce patient anxiety. The elements of the design and how they function are described below. This application claims the benefit of provisional application Serial No. 61/235,278 filed August 19, 2009. The devices US 6 830 560 and WO 2009/040602 disclose automatic injection devices. comprise a prefilled syringe with an attached needle and needle stick safety devices.

### BRIEF DESCRIPTION OF THE DRAWINGS

Figure 1 illustrates the fully assembled device;
Figure 2 illustrates the fully assembled device quarter section view;
Figure 3 illustrates the exploded view of various components of the device;
Figure 4 illustrates the needle guard body grooves showing a position of the main body protrusions during the steps of operating the device;
Figure 5 illustrates rigid needle shield (RNS) removal;
Figure 6 illustrates the device ready for medication dispensing;
Figure 7 illustrates medication dispensing steps;
Figure 8 is a cross section after an injection of medication;
Figure 9 illustrates removal of the device and needle from the injection site;
Figure 10 illustrates the final safety configuration;
Figure 11 illustrates the main body window position prior to the removal of the RNS and RNS Removal Tool;
Figure 12 provides an illustration after the RNS has been removed and the needle guard body has moved forward;
Figure 13 is a detailed showing of the RNS removal tool improvement;
Figure 14 illustrates the RNS removal tool radial CAM mechanism to engage RNS;
Figure 15 illustrates the RNS removal tool axial CAM mechanism;
Figure 16 shows the RNS removal tool retention tool, nominal orientation allowing syringe assembly; and
Figure 17 shows the removal tool retention barb engaging proximal end of the RNS.

### DETAILED DESCRIPTION

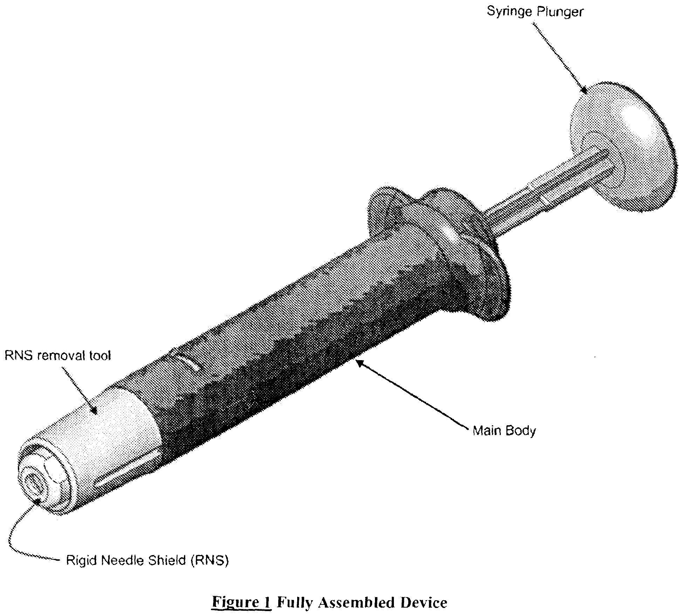

### Figure 1 Fully Assembled Device

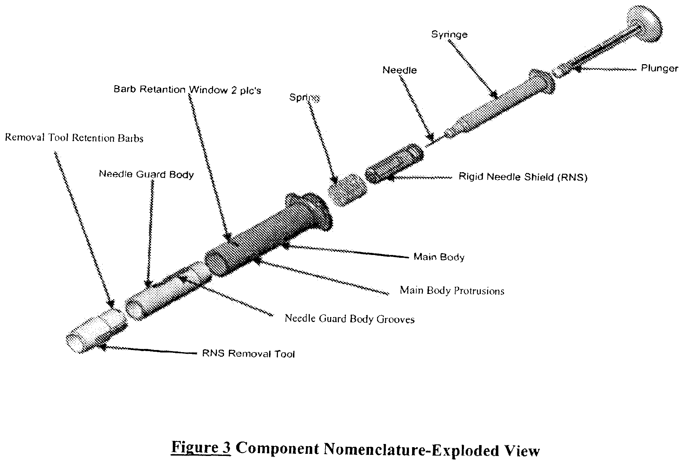

The fully assembled device is shown in Figure 1. The assembly comprises of a Main Body with a Plunger emanating proximally from the proximal end. Inside the device is a prefilled syringe with an attached needle and a cover over the needle called a Rigid Needle Shield (RNS) that protects the needle and maintains a sterile barrier for the medication injection pathways. At the distal end of the assembly is the RNS Removal Tool, which is a removable covering over the RNS that facilitates its removal just before injecting medication into the patient. The plunger is attached to the syringe stopper. At the distal end of the assembly inside the Main Body is the Needle Guard Body, which is a tube that is concentric and interior to the Main Body (see Figures 3, 5, and 6).

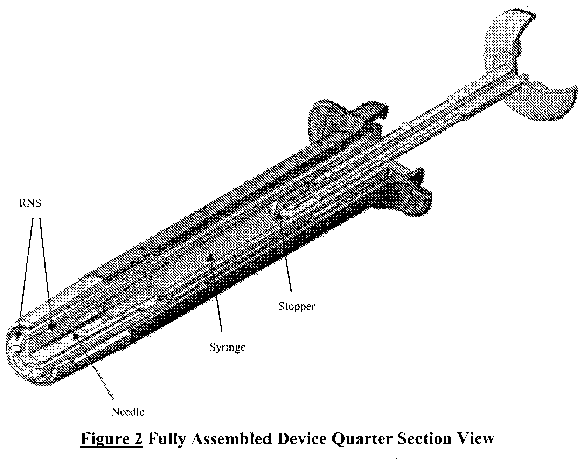

### Figure 2 Fully Assembled Device Quarter Section View

The Needle Guard Body is axially slide-able with respect to the rest of the Main Body and is biased in a distal direction by a compression spring acting at its proximal end. Initially, the Needle Guard Body is held in a proximal position by the RNS Removal Tool. The RNS Removal Tool is held in this position against the force of the spring by Retention Barbs that project outwardly at the proximal end of the RNS Removal Tool and that mate with corresponding Retention Windows in the wall of the Main Body of the device.

Immediately before injecting medication into a patient, the RNS is removed by squeezing the RNS Removal Tool, which collapses along two slits that run along the side of the tool starting at its proximal end. The collapsed configuration of the tool allows the Retention Barbs at the proximal end to disengage from the corresponding Retention Windows in the Main Body of the device. Inwardly projecting RNS Barbs at the proximal inside surface of the RNS Removal Tool grasp the proximal edge of the RNS, which in combination with the compressive force transmitted by the collapsed RNS Removal Tool walls allows it to pull the RNS from the distal end of the syringe when the user pulls it in a distal direction.

### Figure 3 Component Nomenclature-Exploded View

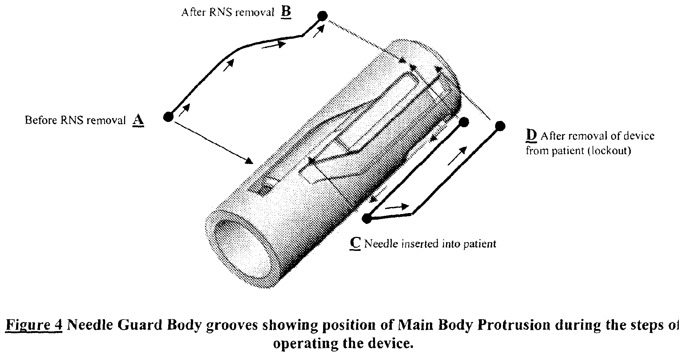

As the RNS Removal Tool is withdrawn from the end of the device, the Needle Guard Body slides forward to an intermediate stop point governed by the interference between one or more inwardly projecting Protrusions from the Main Body and corresponding grooves in the outer surface of the Needle Guard Body (see Figure 4). The outer side of one Protrusion on the Main Body is shown in Figure 3. They are positioned at the end of cut-out sections that provide flexibility to the Protrusions. The Protrusions interfere with the Needle Guard Body grooves by projecting into the groove space and controlling the movement of the Needle Guard Body against the distally directed force of the spring and the proximally directed reaction force from the patient's skin. Prior to removal of the RNS, the Protrusion is in position A of the Needle Guard Body groove as shown in Figure 4. After the RNS is removed, the Needle Guard Body moves distally in response to the spring force, so that the Protrusion is at position B. As the needle is inserted into the patient, the patient's skin pushes the Needle Guard Body proximally against the spring force, such that the groove-protrusion interface moves from position B to position C. Toward the distal end of this groove section, the groove depth steps down to a deeper portion along an edge that is angled to the axis of the needle guard body. After injection is complete and the device is pulled away from the patient, the Needle Guard Body moves distally from position C such that the Protrusions encounter the stepped angled edge groove under the force of the spring. The angled stepped surface causes the Needle Guard Body to rotate with respect to the Main Body and to enter a final groove toward position D, wherein the Protrusion drops into a further deepening of the groove such that the protrusion is substantially captured. This engagement prevents relative motion of the Needle Guard Body with respect to the Main Body

At this point the Needle Guard Body has projected distally around the needle to the extent that it protects the caregiver and others from inadvertently being stuck by the needle tip. The Needle Guard Body is held in this needle-shielding position by interference of the Protrusions in the groove recess at position D so that the Needle Guard Body can not be pushed proximally with respect to the Main Body.
Figure 4 Needle Guard Body grooves showing position of Main Body Protrusion during the steps of operating the device.

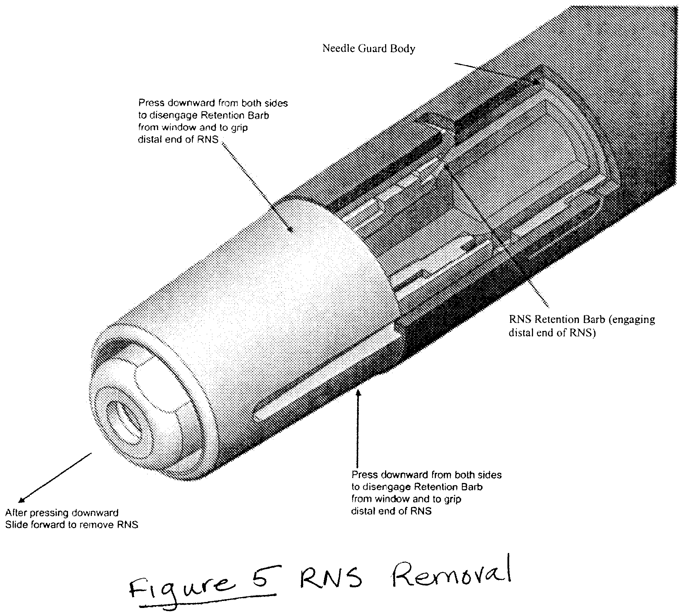

Figure 5 RNS Removal

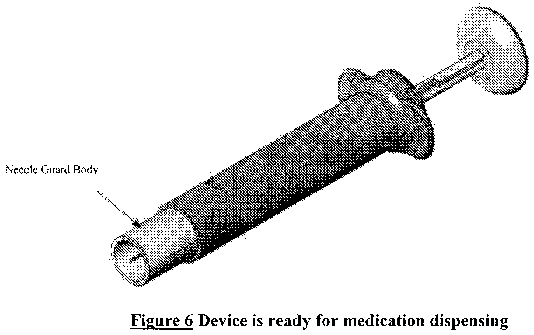

Figure 6 Device is ready for medication dispensing

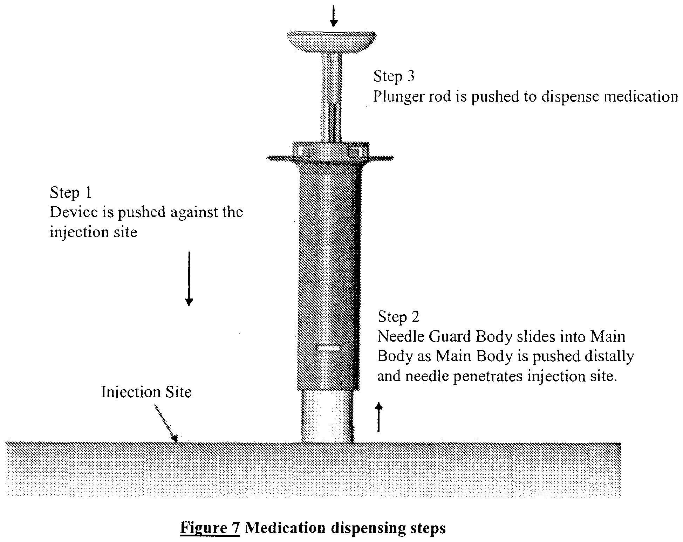

Figure 7 Medication dispensing steps

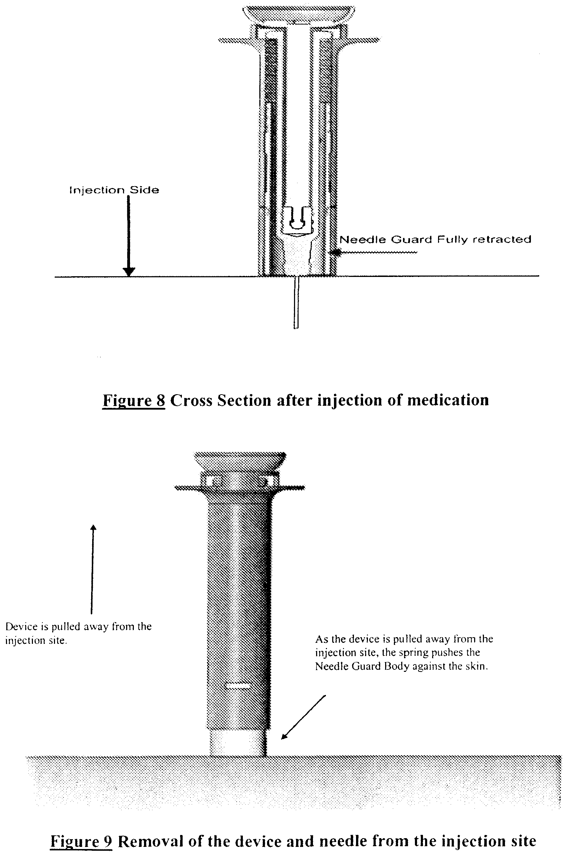

Figure 8 Cross Section after injection of medication
Figure 9 Removal of the device and needle from the injection site

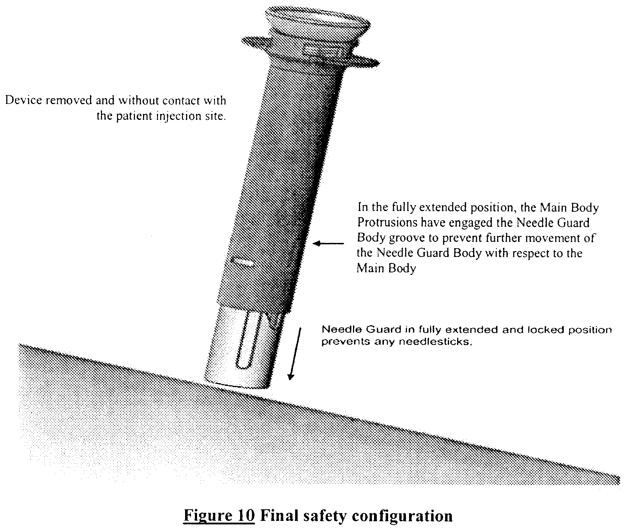

Figure 10 Final safety configuration

The sequence of steps to operate the device is described in Figures 5 through 10. The RNS Removal Tool is squeezed and pulled distally to remove the RNS as shown in Figure 5. As a result of this, the Needle Guard Body moves distally to the position shown in Figure 6. A composite of 3 steps to inject medication are shown in Figure 7. In Step 1 the device is pushed against the patient's injection site. In Step 2, as the device is pushed against the injection site, the Needle Guard Body moves proximally allowing the needle to enter into the injection site. In Step 3, the Plunger Rod is pushed forward to dispel the medication into the injection site. Figure 8 shows a cross-section of the device after the plunger has been fully depressed. In Figure 9, the device is being withdrawn from the injection site while the spring pushes the Needle Guard Body distally. As the device is fully withdrawn from the injection site, the Needle Guard Body fully extends forward as shown in Figure 10. At this point the Main Body Protrusions have entered position D in Figure 4 and locked the Needle Guard Body from further motion.

To facilitate movement of the Main Body and its Protrusions with respect to the Grooves of the Needle Guard Body, one or both components can be made using a plastic resin with ample lubrication (e.g. high content of mold release). Alternatively, dissimilar plastic resins exhibiting a low mutual coefficient of friction can be used for the components.

It is to be understood that there exist alternative arrangements of components that would still fall within the scope of what is described and claimed within this application. For instance, the Needle Guard Body could be positioned on the outside of the main body with interior-facing grooves and outwardly facing protrusions on the main body.

### Light Protected Embodiment

An alternative embodiment for the safety device is presented for use with light-sensitive drugs that require only minimal exposure to light. In this embodiment, the Patient-Contact Activated Needle Stick Safety Device components are made of opaque materials (e.g. plastic resins with pigments, tinted glass, etc.) that effectively block light from reaching the drug in the medication delivery device. However, drug injection instructions normally require the caregiver to inspect the drug to check that it is not cloudy, etc. prior to giving the injection. To achieve this, the Main Body and Needle Guard Body of the device each have diametrically opposed windows that are positioned with respect to each other such that they are not aligned until the RNS Removal Tool and RNS have been removed. After removal, when the diametrically opposed windows on the two components align, they form a line of sight through the device, which enables the caregiver to inspect the drug volume. The RNS, RNS Removal Tool, and Plunger rod components would also be made of opaque materials to prevent light exposure at the ends of the device. A covering (not shown) over the proximal end of the syringe with a hole for the plunger rod could also be created to provide additional light protection.

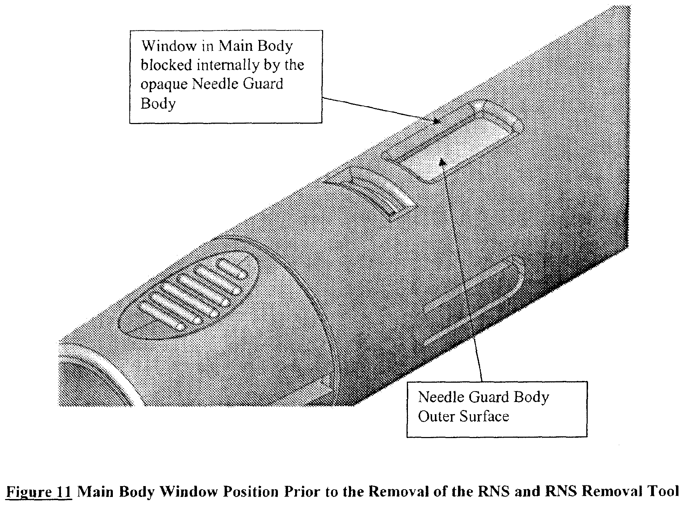

Figure 11 Main Body Window Position Prior to the Removal of the RNS and RNS Removal Tool

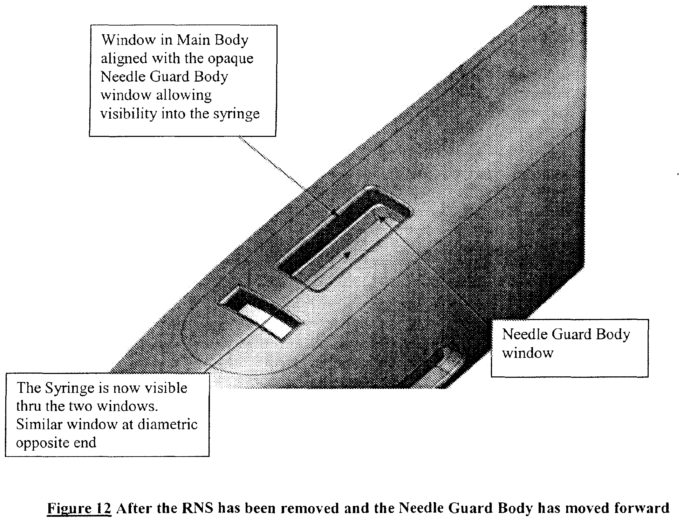

Figure 12 After the RNS has been removed and the Needle Guard Body has moved forward.

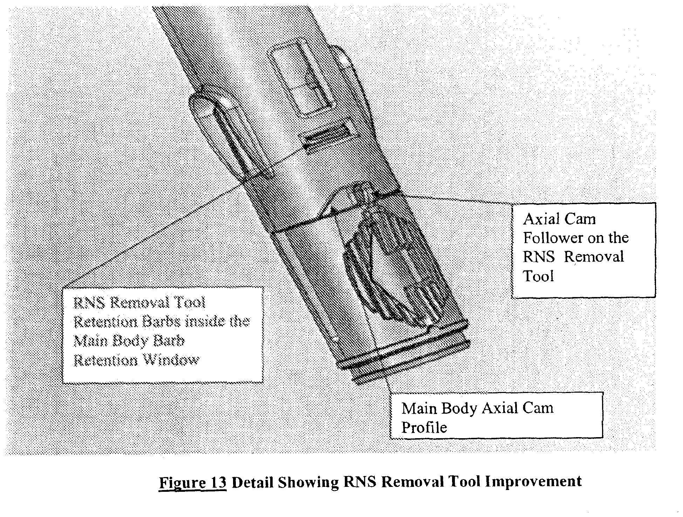

Figure 13 Detail Showing RNS Removal Tool Improvement

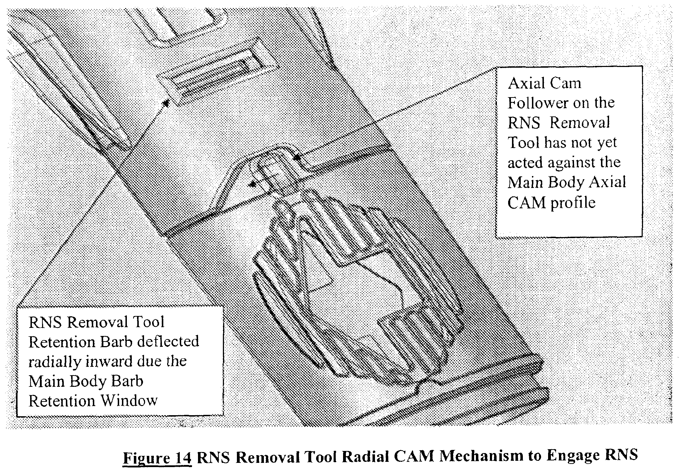

Figure 14 RNS Removal Tool Radial CAM Mechanism to Engage RNS

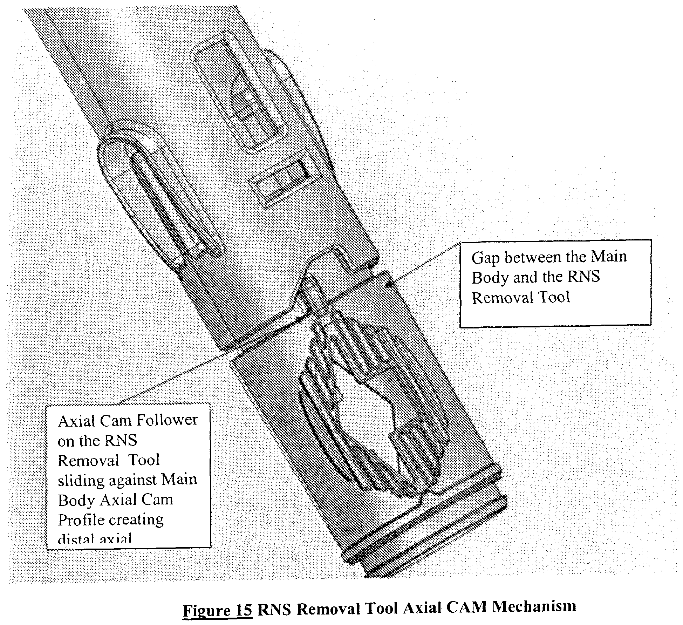

Figure 15 RNS Removal Tool Axial CAM Mechanism

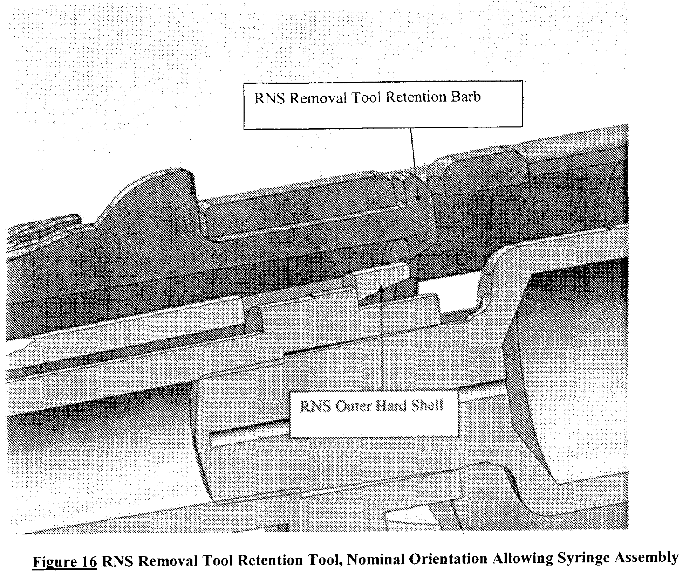

Figure 16 RNS Removal Tool Retention Tool, Nominal Orientation Allowing Syringe Assembly

The Rigid Needle Shield not only protects the needle from being bent or its tip from being damaged but it also forms one of the sterile barriers for the drug closure system. It must perform these functions before, during, and after sterilization and is therefore a complicated component that receives a tremendous amount of testing during drug development and approval process. Since it has potential contact with the drug inside the syringe, it becomes part of the specific drug closure system that receives regulatory approval and is therefore difficult to change after approval. They have become industry standard devices produced by specialized third party manufacturers. Nevertheless, they have limitations and deficiencies, namely that they can become difficult to remove from the syringe after sterilization and storage, often requiring greater than 20N of force to remove, which on a part so small (approximately 0.25 inches in diameter, 1 inch long) makes it difficult for healthcare workers to remove due to the small grasping area. Patients that perform self-administration, especially those with limited manual dexterity or strength (e.g. arthritic or multiple sclerosis patients) will find it extremely difficult to remove. Therefore, an added improvement of the present device is to facilitate the RNS removal. This is accomplished by the RNS Removal Tool, which in addition to presenting a bigger surface area with which the user can grab, it also features some CAM mechanisms to provide a mechanical leverage to removing the RNS. As shown in Figures 13, 14, and 16 the RNS Removal Tool Retention Barbs reside inside the Main Body Barb Retention Window before the RNS is removed. The lateral sides of this Barb and or the corresponding edges of the Main Body Barb Retention Window are angled such that as the RNS removal tool is rotated, the barbs push against the edge of the window and are deflected radially inward as shown in Figures 14 and 15.

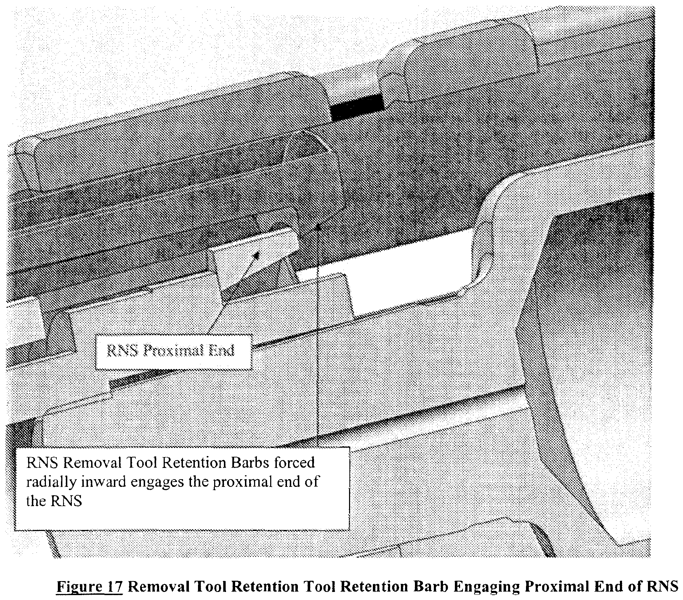

### Figure 17 Removal Tool Retention Tool Retention Barb Engaging Proximal End of RNS

This mechanical advantage provides a strong radial squeeze so that the removal tool further engages the proximal edge of the RNS as shown in Figure 17. This engagement cannot pre-exist sufficiently since the syringe and RNS must assemble into the device, from the proximal to distal end of device, with minimal resistance or disturbance to the RNS seal as shown in Figure 16. After the proximal end of the RNS is engaged by the RNS Removal Tool, the Axial Cam Follower engages a sloped surface, the Main Body Axial Cam Profile as shown in Figures 15 and 17, which places a mechanically advantaged axial force on the RNS Removal Tool in a distal direction. Because the proximal end of the RNS is engaged, the RNS Removal Tool pushes the RNS off of the syringe and needle with much less effort on the part of the user than would normally be required.

Although this description has used a Rigid Needle Shield as an example, soft needle shields, which do not have a hard plastic outer shell, could equally be used in this application with minor changes to account for different geometry.

A further improvement to the device could be a distal end cap on the RNS Removal Tool and a proximal inwardly projecting lip that together would help contain the RNS after it had been removed from the syringe, preventing it from falling to the floor, etc.

The RNS Removal Tool can also have large cut-through arrows indicating the direction of rotation to the end user. It could also have large wings extending radially outward to provide greater rotational mechanical advantage for the end user.

For patients that have limited hand strength, holding the device against the skin while the needle is in the injection site, requires maintaining a force against the spring that pushes against the Needle Guard Body (position C in Figure 4). A further improvement to the device would be to lessen this force by increasing the angle of the deeper groove section that starts proximally to point C in Figure 4 and deflects the Main Body Protrusion over to the straight groove section that ends at D. In Figure 4, this angle is shown at about 45 degrees. An angle of perhaps 60 degrees would place a greater axial component of force against the spring force at some reduction of the lateral force.

Of course, an angle of 90 degrees would hold the Needle Guard Body completely against the force of the spring if the Main Body Protrusion could stay down in the deeper section of the groove, but there would be no lateral deflection to get the Main Body Protrusion over to point D where the device locks out into the desired safety configuration.

Depending on the coefficient of friction between the Main Body Protrusion and the Needle Guard Body, the angle can be optimized to reduce the holding force for the patient, but still allow the Main Body Protrusion to lockout at point D of Figure 4.

The glass syringe and rubber stopper have for years provided an ideal drug storage closure having unique properties of impermeability to oxygen, low extractables, biocompability, durability, etc. However they are both formed by processes that do not lend themselves to tight geometrical tolerances. For instance, the syringe flange is formed when a glass tube is heated to a soft state and the edges pressed over to form an edge. Typical tolerances for the inside length of a syringe or the length of a stopper are both +/- 0.5 mm. The finger flange thickness has a similar tolerance. Furthermore, tight tolerances were not originally needed by these devices because they were not used mechanically with other devices. Existing passive anti-needle stick safety devices for prefilled syringes must mount to the syringe but not interfere excessively with the force required to move the plunger rod during injection nor prevent the full travel of the plunger rod. The safety mechanism necessarily must be triggered toward the end of administration of the drug (near the end of the plunger rod travel). However, since virtually all safety devices locate the syringe against the safety device at a point under the syringe finger flange, a stackup of worst-case tolerances can put the required plunger rod travel variance at +/- 1.5 mm when considering just the tolerances of the inside length of the syringe, syringe flange thickness, and stopper length (syringe manufacturers reference the syringe length from the proximal end of the syringe, not the distal underside of the finger flange). To accommodate this 3 mm range of plunger rod position variance is very difficult for safety devices and it is a deliberate aim of the present invention to reduce and or eliminate any dependence of the safety device on the syringe and stopper tolerances. This is accomplished by having the safety device triggering mechanism independent of the syringe geometry. The present device is triggered when the Needle Guard Body is displaced proximally as the needle is inserted into the patient. The triggering point is broadly placed between point C in Figure 4 and the angled step down feature proximal to point C. As long as the Needle Guard Body is pushed such that the Main Body Protrusion makes it to anywhere between point C and the angled step down, the device will lockout and this is almost completely independent of the syringe or stopper geometry.

The present safety device also makes the needle shielding completely contemporaneous with needle removal, reducing the possibility of needle stick injuries when, for instance, a patient suddenly jerks or flinches causing the needle to be come out of the patient before the plunger rod had fully traveled and activated the safety mechanism as would be the case with existing passive safety devices.

## Claims

1. A needle stick safety device for a pre-filled syringe with an attached needle and a rigid needle shield (RNS) over the needle, the needle stick safety device being intended to prevent needle stick injuries after medication has been injected, and comprising:
- a main body for receiving the syringe, the main body comprising protrusions,
- a RNS removal tool removably mounted at the distal end of the device, the RNS removal tool forming a removable covering over the rigid needle shield for facilitating removal of said rigid needle shield just before injecting medication to a patient,
- a needle guard body forming a tube that is concentric and interior or exterior to the main body, the needle guard body being slidable with respect to the main body and being biased in the distal direction by a compression spring, the needle guard body including grooves with which the protrusions of the main body interfere in order to control the movement of the needle guard body with respect to the main body,
wherein:
- from a configuration in which the device is ready for medication dispensing, as the device is pushed against the injection site, the needle guard body moves proximally, against the spring force, allowing the needle to enter into the injection site, the groove-protrusion interface moving from a second position (B) to a third position (C);
- when the device is being withdrawn from the injection site, the spring pushes the needle guard body distally towards a fully forward extended position, the protrusions of the main body moving in the grooves of the needle guard body from the third position (C) to a fourth position (D) in which the needle guard body is locked from further motion;
**characterized in that**, in order to place the device in the configuration in which it is ready for medication dispensing, the RNS removal tool is removed from the end of the device, thereby removing the rigid needle shield and causing the needle guard body to move distally in response to the spring force, so that the protrusions of the main body move in the grooves of the needle guard body from a first position (A) to the second position (B).

2. A needle stick safety device as in claim 1 wherein the main body includes barb retention windows for engaging barbs on the RNS removal tool.

3. A needle stick safety device as in claim 2, wherein the retention barbs project outwardly at the proximal end of the RNS removal tool and mate with the barb retention windows in the wall of the main body, whereby the RNS removal tool and the needle guard body are held in a proximal position against the force of the spring.

4. A needle stick safety device as in any one of claims 1 to 3, wherein the RNS removal tool comprises two slits that run along its side starting at its proximal end, whereby the RNS removal tool can be squeezed and can collapse along said slits.

5. A needle stick safety device as in any one of claims 1 to 4, wherein the RNS removal tool comprises inwardly projecting RNS barbs at its proximal inside surface, configured to grasp the proximal edge of the rigid needle shield, so that pulling the RNS removal tool in the distal direction causes pulling the rigid needle shield from the distal end of the syringe.

6. A needle stick safety device as in any one of claims 1 to 5, wherein, toward the distal end of the groove section from the second position (B) to the third position (C), the groove depth steps down to a deeper portion along an edge that is angled to the axis of the needle guard body.

7. A needle stick safety device as in claim 6, wherein interference between the protrusions and said angled edge, under the force of the spring, causes the needle guard body to rotate with respect to the main body and to enter a section of the groove between the third position (C) and the fourth position (D).

8. A needle stick safety device as in any one of claims 1 to 7, wherein, towards the fourth position (D), the groove further deepens, such that the protrusions can be substantially captured.

9. A needle stick safety device as in any one of claims 1 to 8, wherein the main body and needle guard body are made of opaque materials and have diametrically opposed windows that are positioned with respect to each other such that:
- they are not aligned until the RNS removal tool and rigid needle shield have been removed,
- and they are aligned once the RNS removal tool and rigid needle shield have been removed, to form a line of sight through the device.

10. A needle stick safety device as in any one of claims 1 to 9, further including some cam mechanisms to provide a mechanical leverage to removing the rigid needle shield, such as a radial cam mechanism or an axial cam mechanism.

11. A needle stick safety device as in any one of claims 2 to 10, wherein the lateral sides of the barbs on the RNS removal tool and/or the corresponding edges of the barb retention windows of the main body are angled such that, as the RNS removal tool is rotated, the barbs push against the edge of the window and are deflected radially inwardly.

12. A needle stick safety device as in any one of claims 1 to 11, wherein the RNS removal tool is configured to engage the proximal end of the rigid needle shield, and wherein the main body comprises an axial cam profile forming a sloped surface, and the RNS removal tool comprises an axial cam follower configured for engaging said sloped surface.

13. A syringe assembly comprising:
- a pre-filled syringe with an attached needle and a rigid needle shield (RNS) over the needle,
- a needle stick safety device as in any one of claims 1 to 12.

## Patentansprüche

1. Nadelstich-Sicherheitsvorrichtung für eine vorgefüllte Spritze mit einer angebauten Nadel und einem starren Nadelschirm (SNS) über der Nadel, wobei die Nadelstich-Sicherheitsvorrichtung dazu bestimmt ist, Nadelstichverletzungen zu verhindern, nachdem ein Medikament injiziert wurde, und umfasst:
- einen Hauptkörper zum Aufnehmen der Spritze, wobei der Hauptkörper Vorsprünge umfasst,
- ein SNS-Abnahmewerkzeug, das abnehmbar am distalen Ende der Vorrichtung angebracht ist, wobei das SNS-Abnahmewerkzeug eine abnehmbare Abdeckung über dem starren Nadelschirm bildet, um das Abnehmen des starren Nadelschirms unmittelbar vor dem Injizieren eines Medikaments in einen Patienten zu erleichtern,
- einen Nadelschutzkörper, der ein Rohr bildet, das zum Hauptkörper konzentrisch ist und sich innerhalb oder außerhalb desselben befindet, wobei der Nadelschutzkörper in Bezug auf den Hauptkörper verschiebbar ist und durch eine Kompressionsfeder in der distalen Richtung vorgespannt ist, wobei der Nadelschutzkörper Nuten einschließt, mit denen die Vorsprünge des Hauptkörpers zusammenwirken, um die Bewegung des Nadelschutzkörpers in Bezug auf den Hauptkörper zu steuern,
wobei:
- wenn ausgehend von einer Konfiguration, in der die Vorrichtung zur Medikamentenabgabe bereit ist, die Vorrichtung gegen die Injektionsstelle gedrückt wird, sich der Nadelschutzkörper entgegen der Federkraft proximalwärts bewegt, was es der Nadel ermöglicht, in die Injektionsstelle einzudringen, wobei sich die Nut-/Vorsprungs-Schnittstelle von einer zweiten Position (B) zu einer dritten Position (C) bewegt;
- wenn die Vorrichtung von der Injektionsstelle zurückgezogen wird, die Feder den Nadelschutzkörper distalwärts zu einer vollständig nach vorne gestreckten Position drückt, wobei sich die Vorsprünge des Hauptkörpers in den Nuten des Nadelschutzkörpers von der dritten Position (C) zu einer vierten Position (D) bewegen, in der der Nadelschutzkörper gegen weitere Bewegung gesperrt ist;
**dadurch gekennzeichnet, dass**, um die Vorrichtung in die Konfiguration zu versetzen, in der sie zur Medikamentenabgabe bereit ist, das SNS-Abnahmewerkzeug vom Ende der Vorrichtung abgenommen wird, wodurch der starre Nadelschirm abgenommen und bewirkt wird, dass sich der Nadelschutzkörper in Reaktion auf die Federkraft distalwärts bewegt, sodass sich die Vorsprünge des Hauptkörpers in den Nuten des Nadelschutzkörpers von einer ersten Position (A) zur zweiten Position (B) bewegen.

2. Nadelstich-Sicherheitsvorrichtung nach Anspruch 1, wobei der Hauptkörper Hakenhaltefenster zum Ineinandergreifen mit Haken am SNS-Abnahmewerkzeug umfasst.

3. Nadelstich-Sicherheitsvorrichtung nach Anspruch 2, wobei die Haltehaken am proximalen Ende des SNS-Abnahmewerkzeugs nach außen vorstehen und sich in die Hakenhaltefenster in der Wand des Hauptkörpers einpassen, wodurch das SNS-Abnahmewerkzeug und der Nadelschutzkörper entgegen der Kraft der Feder in einer proximalen Position gehalten werden.

4. Nadelstich-Sicherheitsvorrichtung nach einem der Ansprüche 1 bis 3, wobei das SNS-Abnahmewerkzeug zwei Schlitze umfasst, die an seinem proximalen Ende beginnend seiner Seite entlang verlaufen, wodurch das SNS-Abnahmewerkzeug zusammengedrückt werden und entlang der Schlitze einknicken kann.

5. Nadelstich-Sicherheitsvorrichtung nach einem der Ansprüche 1 bis 4, wobei das SNS-Abnahmewerkzeug an seiner proximalen Innenfläche nach innen vorstehende SNS-Haken umfasst, die dazu konfiguriert sind, die proximale Kante des starren Nadelschirms zu greifen, sodass das Ziehen des SNS-Abnahmewerkzeugs in der distalen Richtung das Ziehen des starren Nadelschirms vom distalen Ende der Spritze bewirkt.

6. Nadelstich-Sicherheitsvorrichtung nach einem der Ansprüche 1 bis 5, wobei zum distalen Ende des Nutabschnitts von der zweiten Position (B) zur dritten Position (C) hin die Nuttiefe zu einem tieferen Teil entlang einer Kante zurückspringt, der zur Achse des Nadelschutzkörpers abgewinkelt ist.

7. Nadelstich-Sicherheitsvorrichtung nach Anspruch 6, wobei das Zusammenwirken zwischen den Vorsprüngen und der abgewinkelten Kante unter der Kraft der Feder bewirkt, dass sich der Nadelschutzkörper in Bezug auf den Hauptkörper dreht und in einen Abschnitt der Nut zwischen der dritten Position (C) und der vierten Position (D) eindringt.

8. Nadelstich-Sicherheitsvorrichtung nach einem der Ansprüche 1 bis 7, wobei sich die Nut zur vierten Position (D) hin weiter vertieft, derart, dass die Vorsprünge im Wesentlichen gefasst werden können.

9. Nadelstich-Sicherheitsvorrichtung nach einem der Ansprüche 1 bis 8, wobei der Hauptkörper und Nadelschutzkörper aus undurchsichtigen Materialien gefertigt sind und diametral gegenüberliegende Fenster aufweisen, die in Bezug zueinander derart positioniert sind, dass:
- sie nicht fluchten, bis das SNS-Abnahmewerkzeug und der starre Nadelschirm abgenommen wurden,
- und sie fluchten, sobald das SNS-Abnahmewerkzeug und der starre Nadelschirm abgenommen wurden, um eine Sichtlinie durch die Vorrichtung zu bilden.

10. Nadelstich-Sicherheitsvorrichtung nach einem der Ansprüche 1 bis 9, weiter einige Nockenmechanismen wie etwa einen radialen Nockenmechanismus oder einen axialen Nockenmechanismus einschließend, um eine mechanische Hebelwirkung zum Abnehmen des starren Nadelschirms bereitzustellen.

11. Nadelstich-Sicherheitsvorrichtung nach einem der Ansprüche 2 bis 10, wobei die lateralen Seiten der Haken am SNS-Abnahmewerkzeug und/oder die entsprechenden Kanten der Hakenhaltefenster des Hauptkörpers derart abgewinkelt sind, dass die Haken gegen die Kante des Fensters drücken und radial einwärts abgelenkt werden, wenn das SNS-Abnahmewerkzeug gedreht wird.

12. Nadelstich-Sicherheitsvorrichtung nach einem der Ansprüche 1 bis 11, wobei das SNS-Abnahmewerkzeug dazu konfiguriert ist, mit dem proximalen Ende des starren Nadelschirms ineinanderzugreifen, und wobei der Hauptkörper ein axiales Nockenprofil umfasst, das eine schräge Fläche bildet, und das SNS-Abnahmewerkzeug einen axialen Nockenfolger umfasst, der dafür konfiguriert ist, mit der schrägen Fläche ineinanderzugreifen.

13. Spritzenbaugruppe, umfassend:
- eine vorgefüllte Spritze mit einer angebauten Nadel und einem starren Nadelschirm (SNS) über der Nadel,
- eine Nadelstich-Sicherheitsvorrichtung nach einem der Ansprüche 1 bis 12.

## Revendications

1. Dispositif de sécurité contre les piqûres d'aiguille pour une seringue préremplie avec une aiguille attachée et une protection rigide d'aiguille (RNS) sur l'aiguille, le dispositif de sécurité contre les piqûres d'aiguille étant destiné à éviter les blessures par piqûre d'aiguille après l'injection d'un médicament, et comprenant :
- un corps principal pour recevoir la seringue, le corps principal comprenant des saillies,
- un outil de retrait de RNS monté de manière amovible au niveau de l'extrémité distale du dispositif, l'outil de retrait de RNS formant un revêtement amovible sur la protection rigide d'aiguille pour faciliter le retrait de ladite protection rigide d'aiguille juste avant l'injection d'un médicament à un patient,
- un corps de gaine d'aiguille formant un tube qui est concentrique et intérieur ou extérieur au corps principal, le corps de gaine d'aiguille pouvant coulisser par rapport au corps principal et étant sollicité dans la direction distale par un ressort de compression, le corps de gaine d'aiguille comportant des rainures avec lesquelles interfèrent les saillies du corps principal afin de commander le mouvement du corps de gaine d'aiguille par rapport au corps principal, dans lequel :
- à partir d'une configuration dans laquelle le dispositif est prêt pour la distribution de médicament, lorsque le dispositif est poussé contre le site d'injection, le corps de gaine d'aiguille se déplace de manière proximale, contre de la force du ressort, permettant à l'aiguille d'entrer dans le site d'injection, l'interface rainure-saillie se déplaçant d'une deuxième position (B) à une troisième position (C) ;
- lorsque le dispositif est retiré du site d'injection, le ressort pousse le corps de gaine d'aiguille de manière distale vers une position complètement déployée vers l'avant, les saillies du corps principal se déplacent dans les rainures du corps de gaine d'aiguille de la troisième position (C) à une quatrième position (D) dans laquelle le corps de gaine d'aiguille est empêché de se déplacer davantage ;
**caractérisé en ce que**, afin de placer le dispositif dans la configuration dans laquelle il est prêt pour la distribution de médicament, l'outil de retrait de RNS est retiré de l'extrémité du dispositif retirant ainsi la protection rigide d'aiguille et amenant le corps de gaine d'aiguille à se déplacer de manière distale en réponse à la force du ressort, de sorte que les saillies du corps principal se déplacent dans les rainures du corps de gaine d'aiguille d'une première position (A) à la deuxième position (B).

2. Dispositif de sécurité contre les piqûres d'aiguille selon la revendication 1, dans lequel le corps principal comporte des fenêtres de rétention d'ergots pour venir en prise avec des ergots sur l'outil de retrait de RNS.

3. Dispositif de sécurité contre les piqûres d'aiguille selon la revendication 2, dans lequel les ergots de rétention font saillie vers l'extérieur au niveau de l'extrémité proximale de l'outil de retrait de RNS et s'accouplent avec les fenêtres de rétention d'ergots dans la paroi du corps principal, moyennant quoi l'outil de retrait de RNS et le corps de gaine d'aiguille sont maintenus dans une position proximale contre la force du ressort.

4. Dispositif de sécurité contre les piqûres d'aiguille selon l'une quelconque des revendications 1 à 3, dans lequel l'outil de retrait de RNS comprend deux fentes qui s'étendent le long de son côté en commençant à son extrémité proximale, moyennant quoi l'outil de retrait de RNS peut être comprimé et peut se replier le long desdites fentes.

5. Dispositif de sécurité contre les piqûres d'aiguille selon l'une quelconque des revendications 1 à 4, dans lequel l'outil de retrait de RNS comprend des ergots de RNS faisant saillie vers l'intérieur au niveau de sa surface interne proximale, configurés pour saisir le bord proximal de la protection rigide d'aiguille, de sorte que l'extraction de l'outil de retrait de RNS dans la direction distale provoque l'extraction de la protection rigide d'aiguille de l'extrémité distale de la seringue.

6. Dispositif de sécurité contre les piqûres d'aiguille selon l'une quelconque des revendications 1 à 5, dans lequel, en direction de l'extrémité distale de la section de rainure de la deuxième position (B) à la troisième position (C), la profondeur de rainure descend jusqu'à une partie plus profonde le long d'un bord qui est incliné par rapport à l'axe du corps de gaine d'aiguille.

7. Dispositif de sécurité contre les piqûres d'aiguille selon la revendication 6, dans lequel une interférence entre les saillies et ledit bord incliné, sous la force du ressort, amène le corps de gaine d'aiguille à tourner par rapport au corps principal et à pénétrer dans une section de la rainure entre la troisième position (C) et la quatrième position (D).

8. Dispositif de sécurité contre les piqûres d'aiguille selon l'une quelconque des revendications 1 à 7, dans lequel, en direction de la quatrième position (D), la rainure s'approfondie davantage, de sorte que les saillies puissent être sensiblement capturées.

9. Dispositif de sécurité contre les piqûres d'aiguille selon l'une quelconque des revendications 1 à 8, dans lequel le corps principal et le corps de gaine d'aiguille sont réalisés en matériaux opaques et ont des fenêtres diamétralement opposées qui sont positionnées l'une par rapport à l'autre de sorte :
- qu'elles ne soient pas alignées tant que l'outil de retrait de RNS et la protection rigide d'aiguille n'ont pas été retirés,
- et qu'elles soient alignées une fois que l'outil de retrait de RNS et la protection rigide d'aiguille ont été retirés, pour former une ligne de vision à travers le dispositif.

10. Dispositif de sécurité contre les piqûres d'aiguille selon l'une quelconque des revendications 1 à 9, comportant en outre quelques mécanismes à came pour fournir un levier mécanique afin de retirer la protection rigide d'aiguille, tels qu'un mécanisme à came radiale ou un mécanisme à came axiale.

11. Dispositif de sécurité contre les piqûres d'aiguille selon l'une quelconque des revendications 2 à 10, dans lequel les côtés latéraux des ergots sur l'outil de retrait de RNS et/ou les bords correspondants des fenêtres de rétention d'ergots sont inclinés de sorte que, lorsque l'outil de retrait de RNS est tourné, les ergots poussent contre le bord de la fenêtre et sont déviés radialement vers l'intérieur.

12. Dispositif de sécurité contre les piqûres d'aiguille selon l'une quelconque des revendications 1 à 11, dans lequel l'outil de retrait de RNS est configuré pour venir en prise avec l'extrémité proximale de la protection rigide d'aiguille, et dans lequel le corps principal comprend un profil de came axiale formant une surface inclinée, et l'outil de retrait de RNS comprend un suiveur de came axiale configuré pour venir en prise avec ladite surface inclinée.

13. Ensemble seringue comprenant :
- une seringue préremplie avec une aiguille attachée et une protection rigide d'aiguille (RNS) sur l'aiguille,
- un dispositif de sécurité contre les piqûres d'aiguille selon l'une quelconque des revendications 1 à 12.
